(19) Europäisches Patentamt / European Patent Office / Office européen des brevets

(11) **EP 4 416 471 B1**

(12) **EUROPÄISCHE PATENTSCHRIFT**

(45) Veröffentlichungstag und Bekanntmachung des Hinweises auf die Patenterteilung:
**14.05.2025 Patentblatt 2025/20**

(21) Anmeldenummer: **22789830.1**

(22) Anmeldetag: **05.10.2022**

(51) Internationale Patentklassifikation (IPC):
*G01J 3/46* (2006.01)    *D01G 23/08* (2006.01)
*G01N 21/89* (2006.01)    *G01N 33/36* (2006.01)
*G01N 21/84* (2006.01)    *G01N 15/14* (2024.01)

(52) Gemeinsame Patentklassifikation (CPC):
**G01N 15/1456; D01G 23/08; D01G 31/006; G01N 21/8915; G01N 33/362;** G01J 3/46;
G01J 3/462; G01N 15/1433; G01N 2015/1402; G01N 2021/8444; G01N 2021/8592

(86) Internationale Anmeldenummer:
**PCT/CH2022/000008**

(87) Internationale Veröffentlichungsnummer:
**WO 2023/060367 (20.04.2023 Gazette 2023/16)**

(54) **OPTISCHE CHARAKTERISIERUNG EINES TEXTILFASERGEBILDES**

OPTICALLY CHARACTERIZING A TEXTILE FIBER STRUCTURE

CARACTÉRISATION OPTIQUE D'UNE STRUCTURE DE FIBRE TEXTILE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **12.10.2021 CH 0703792021**

(43) Veröffentlichungstag der Anmeldung:
**21.08.2024 Patentblatt 2024/34**

(73) Patentinhaber: **Uster Technologies AG 8610 Uster (CH)**

(72) Erfinder:
• **SIEGENTHALER, Mario 8535 Herdern (CH)**
• **SCHNEIDER, Ulf 8610 Uster (CH)**

(74) Vertreter: **Pliska, Pavel Uster Technologies AG Sonnenbergstrasse 10 8610 Uster (CH)**

(56) Entgegenhaltungen:
EP-A2- 3 321 668    WO-A2-2007/012936
US-A1- 2003 059 090    US-A1- 2019 137 382

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

FACHGEBIET

Die vorliegende Erfindung liegt auf dem Gebiet der Herstellung von Garn. Sie betrifft ein computerimplementiertes Verfahren und eine Vorrichtung zur optischen Charakterisierung eines Textilfasergebildes, gemäss den unabhängigen Patentansprüchen, Sie kann bspw., aber nicht ausschliesslich, in der Putzerei einer Spinnerei zum Einsatz kommen.

STAND DER TECHNIK

Üblicherweise werden Textilfasern in Form von Faserballen in eine Spinnerei geliefert. Dort werden die Textilfaserballen aneinandergereiht, wobei oft absichtlich Textilfaserballen verschiedener Sorten und/oder Qualitäten zu einer einzigen Ballenvorlage zusammengestellt werden, um eine entsprechende Fasermischung zu erhalten. Die Ballenvorlage wird mittels eines Ballenöffners schichtweise abgetragen. Die abgetragenen Textilfasern werden in Form von Faserflocken in einem Luftstrom pneumatisch zu verschiedenen Reinigungsstufen transportiert, welche die Faserflocken feiner auflösen und Fremdmaterialien daraus entfernen.

Die US-6,452,157 B1 befasst sich mit einer Vorrichtung an einer Faserverarbeitungseinrichtung zum Erkennen und Verarbeiten von Verunreinigungen, Fremdstoffen und -fasern in textilem Fasermaterial. Die Vorrichtung hat mindestens zwei Lichtquellen, die das Fasermaterial abwechselnd mit unterschiedlicher Farbe beleuchten. Ausserdem ist ein Sensor vorgesehen, der die Farben des vom Fasermaterial reflektierten Lichtes empfängt. Bei einer sprunghaften Farbänderung des Fasermaterials von einer vorgegebenen Farbe entsteht ein elektrisches Signal. Um eine Anpassung der Leuchtfarben je nach Anwendungsfall, z. B. an unterschiedliche oder wechselnde Farbe des Fasermaterials, zu ermöglichen, wird eine Mehrfarben-Lichtquelle mit mehr als zwei Farben herangezogen. Die Art der Farben der Mehrfarben-Lichtquelle ist in Abhängigkeit von der Farbe des zu beleuchtenden Fasermaterials wählbar.

In einem Verfahren zur Optimierung eines Garnherstellungsprozesses gemäss der WO-2021/051210 A1 werden Fremdmaterialien in einem Textilfasergebilde überwacht. Das Textilfasergebilde wird mit elektromagnetischer Strahlung aus mindestens zwei Farbbereichen angestrahlt. Die Fremdmaterialien werden gemäss ihren Farben in verschiedene Farbklassen klassiert. Bei Vorliegen einer genügend grossen Stichprobe mit klassierten Fremdmaterialien wird für die Farbklassen eine Häufigkeitsverteilung der Fremdmaterialien ermittelt und mit einer Referenz-Häufigkeitsverteilung verglichen. Bei einer Abweichung der ermittelten Häufigkeitsverteilung von der Referenz-Häufigkeitsverteilung wird mindestens eine aus einer Menge von mehreren Optimierungshandlungen vorgenommen, bspw. ein Warnsignal ausgegeben.

Die EP-3'321'68 A2 offenbart ein Verfahren zur Beurteilung der Qualität eines Garns. Das Garn wird mit polychromatischem, vorzugsweise weissem, Licht beaufschlagt. Ein Messsignal, das die roten, grünen und blauen Farbanteile des vom Garn reflektierten Lichtes repräsentiert, wird erfasst. Die roten, grünen und blauen Farbanteile werden in einen Bewertungs-Farbraum transformiert, in dem Messpunkte des Messsignals durch eine erste und eine zweite Koordinate, die die Farbe repräsentieren, und durch eine dritte Koordinate, die die Helligkeit repräsentiert, beschrieben werden. Die Beurteilung der Qualität des Garns erfolgt anhand der ersten, zweiten und dritten Koordinate der Messpunkte in dem Bewertungs-Farbraum. Dadurch ist es möglich, Fremdstoffe von Durchmesserabweichungen des Garns zu unterscheiden. Auch schwarze Fremdfasern können detektiert werden.

Nicht nur Fremdmaterialien sind aber ein Problem in der Spinnerei, sondern auch das textile Grundmaterial an sich. Es kommt immer wieder vor, dass sich dieses während der Produktion mit der Zeit unbeabsichtigterweise verändert. Solche Materialänderungen können verschiedene Gründe haben, z. B. eine fehlerhafte Zusammenstellung der Ballenvorlage oder eine Qualitätsänderung innerhalb eines Loses von Textilfaserballen. Im Gegensatz zum Auftreten eines Fremdmaterials erfolgen sie nicht sprunghaft, sondern kontinuierlich innerhalb eines längeren Zeitraums von mehreren Minuten oder Stunden. Die Materialänderungen schlagen sich in unerwünschten Qualitätsänderungen des hergestellten Garns und schliesslich auch der textilen Endprodukte nieder.

DARSTELLUNG DER ERFINDUNG

Es ist eine Aufgabe der vorliegenden Erfindung, ein Verfahren und eine Vorrichtung zur optischen Charakterisierung eines Textilfasergebildes anzugeben. Mit der Erfindung sollen unerwünschte Qualitätsänderungen des textilen Fasergebildes und der daraus hergestellten textilen Endprodukte vermieden werden. Insbesondere sollen grössere Mengen fälschlicherweise in das Textilfasergebilde gelangten Materials und unbeabsichtigte Änderungen der Ballenvorlage erkannt werden. Die dadurch verursachten Materialänderungen erfolgen kontinuierlich innerhalb eines längeren Zeitraums von mehreren Minuten oder Stunden. Die vorliegende Erfindung ist also nicht primär an Einzelereignissen im Textilfasergebilde wie z. B. am Vorhandensein eines einzelnen Fremdmaterials interessiert, sondern an der mittel- und langfristigen Zusammensetzung des Textilfasergebildes. Eine weitere Aufgabe der Erfindung besteht darin, die Ausscheidung von Fremdmaterialien aus dem Textilfasergebilde zu optimieren.

Diese und andere Aufgaben werden durch das erfindungsgemässe computerimplementierte Verfahren und die erfindungsgemässe Vorrichtung gelöst, wie sie in den unabhängigen Patentansprüchen definiert sind. Vorteilhafte Ausführungsformen sind in den abhängigen Patentansprüchen angegeben.

Das erfindungsgemässe computerimplementierte Verfahren dient zur optischen Charakterisierung eines Textilfasergebildes. Dabei wird eine Vielzahl von Farbinformationen über verschiedene Stellen des Textilfasergebildes von einem optischen Sensorsystem erfasst. Die erfassten Farbinformationen werden vom optischen Sensorsystem an einen Computer gesendet, von diesem empfangen und gespeichert. Die gespeicherten Farbinformationen werden vom Computer in einem Farbraum als Punktewolke eingetragen. Eine Häufigkeitsdichteverteilung der Punktewolke wird vom Computer bestimmt. Die Häufigkeitsdichteverteilung wird vom Computer numerisch spezifiziert. Ein von der numerischen Spezifikation abhängiges Signal wird vom Computer ausgegeben.

Die Erfassung der Farbinformationen erfolgt vorzugsweise stichprobenartig.

Der Farbraum ist z. B. ein ein- oder zweidimensionaler Farbraum, in dem Information über eine Helligkeit unberücksichtigt bleibt.

Gemäss einer Ausführungsform wird vom Computer aufgrund der Häufigkeitsdichteverteilung ein charakteristisches Gebiet in dem Farbraum definiert. Zur numerischen Spezifikation der Häufigkeitsdichteverteilung wird das charakteristische Gebiet numerisch spezifiziert. Zu diesem Zweck kann vom Computer z. B. eine Länge, ein Flächeninhalt bzw. ein Volumen des charakteristischen Gebiets, eine geometrische Form des charakteristischen Gebiets und/oder ein Verlauf einer Grenzlinie bzw. einer Grenzfläche, die das charakteristische Gebiet begrenzt, verwendet werden.

In einem ersten Anwendungsbeispiel wird vom Computer aus einer Änderung der numerischen Spezifikation der Häufigkeitsdichteverteilung auf eine Änderung einer Materialzusammensetzung des Textilfasergebildes geschlossen, und das vom Computer ausgegebene Signal ist von der Änderung der Materialzusammensetzung abhängig. Das vom Computer ausgegebene Signal wird vorzugsweise verwendet, um mindestens eine der folgenden Handlungen auszulösen: Ausgabe einer Grafik an eine Bedienperson, Ausgabe einer Warnung an eine Bedienperson, Ausgabe einer Handlungsempfehlung an eine Bedienperson, Abstellen mindestens einer Verarbeitungsmaschine, Änderung einer Einstellung an mindestens einer Verarbeitungsmaschine. Dem Computer kann ein Grenzwert und/oder ein Toleranzbereich vorgegeben werden, dessen Überschreitung durch einen Parameter der numerischen Spezifikation der Häufigkeitsdichteverteilung eine Änderung der numerischen Spezifikation anzeigt.

In einem zweiten Anwendungsbeispiel wird vom Computer aufgrund der numerischen Spezifikation der Häufigkeitsdichteverteilung ein Fremdmaterial im Textilfasergebilde erkannt, und das vom Computer ausgegebene Signal steuert eine Ausscheidung des erkannten Fremdmaterials aus dem Textilfasergebilde. Dabei kann vom Computer ein Element der Punktewolke als zu einem Fremdmaterial gehörig erkannt werden, wenn das betreffende Element ausserhalb des charakteristischen Gebiets liegt. Gemäss einer ersten Alternative wird vom Computer die Ausscheidung der Fremdmaterialien ausgesetzt, bis ein statistisch repräsentatives charakteristisches Gebiet vorliegt und numerisch spezifiziert ist. Gemäss einer zweiten Alternative wird dem Computer ein initiales charakteristisches Gebiet vorgegeben, und das charakteristische Gebiet wird vom Computer laufend an eine Materialzusammensetzung des Textilfasergebildes angepasst.

Gemäss einer Ausführungsform werden vom Computer die Farbinformationen, ein Schwellenwert und/oder ein Toleranzbereich mit einem Auflösegrad, der angibt, in welchem Masse das Textilfasergebilde in einzelne Faserflocken aufgelöst ist, korrigiert. Zu diesem Zweck wird z. B. vom optischen Sensorsystem mindestens ein Bild des Textilfasergebildes aufgenommen und vom Computer aus dem mindestens einen Bild diejenige Bildfläche, auf die Fasermaterial abgebildet ist, durch Bildverarbeitung bestimmt; vom Computer wird ein Massenfluss des Textilfasergebildes pro Zeiteinheit bestimmt; der Auflösegrad wird vom Computer als skalare Grösse durch Bildung eines Quotienten aus der Bildfläche, auf die Fasermaterial abgebildet ist, einerseits und aus dem Massenfluss andererseits berechnet. Der Auflösegrad wird vom Computer vorzugsweise gemäss der Formel

$$X = A_F / \left( A_T \cdot m \right)$$

bestimmt, worin $A_F$ diejenige Bildfläche, auf die Fasermaterial abgebildet wird, $A_T$ eine Gesamtfläche des Bildes und m einen Massenfluss des Textilfasergebildes pro Zeiteinheit bedeuten.

Die erfindungsgemässe Vorrichtung dient zur optischen Charakterisierung eines Textilfasergebildes. Sie beinhaltet ein optisches Sensorsystem zur Erfassung einer Vielzahl von Farbinformationen über verschiedene Stellen des Textilfasergebildes und einen mit dem optischen Sensorsystem verbundenen Computer. Das optische Sensorsystem weist Sendemittel zum Senden der erfassten Farbinformationen an den Computer auf. Der Computer weist Empfangsmittel zum Empfangen der vom optischen Sensorsystem gesendeten Farbinformationen auf. Der Computer ist dazu eingerichtet, die erfassten Farbinformationen in einem Farbraum als Punktewolke einzutragen, eine Häufigkeitsdichteverteilung der Punktewolke zu bestimmen und die Häufigkeitsdichteverteilung numerisch zu spezifizieren und ein von der numerischen Spezifikation abhängiges Signal auszugeben.

Der Farbraum ist z. B. ein ein- oder zweidimensionaler Farbraum, in dem Information über eine Helligkeit unberücksichtigt bleibt.

Gemäss einer Ausführungsform ist der Computer dazu eingerichtet, aufgrund der Häufigkeitsdichteverteilung ein charakteristisches Gebiet in dem Farbraum zu definieren und zur numerischen Spezifikation der Häufigkeitsdichteverteilung das charakteristische Gebiet numerisch zu spezifizieren.

In einem ersten Anwendungsbeispiel ist der Computer dazu eingerichtet, aus einer Änderung der numerischen Spezifikation der Häufigkeitsdichteverteilung auf eine Änderung einer Materialzusammensetzung des Textilfasergebildes zu schliessen, und das vom Computer ausgegebene Signal ist von der Änderung der Materialzusammensetzung abhängig. Der Computer ist so eingerichtet, dass das von ihm ausgegebene Signal geeignet ist, mindestens eine der folgenden Handlungen auszulösen: Ausgabe einer Grafik an eine Bedienperson, Ausgabe einer Warnung an eine Bedienperson, Ausgabe einer Handlungsempfehlung an eine Bedienperson, Abstellen mindestens einer Verarbeitungsmaschine, Änderung einer Einstellung an mindestens einer Verarbeitungsmaschine.

In einem zweiten Anwendungsbeispiel beinhaltet die Vorrichtung zusätzlich eine mit dem Computer verbundene Ausscheideeinheit. Der Computer ist dazu eingerichtet, aufgrund der numerischen Spezifikation der Häufigkeitsdichteverteilung Fremdmaterialien im Textilfasergebilde zu erkennen und bei Erkennung des Fremdmaterials mittels des von ihm ausgegebenen Signals eine Ausscheidung des erkannten Fremdmaterials durch die Ausscheideeinheit zu steuern. Der Computer kann dazu eingerichtet sein, ein Element der Punktewolke als zu einem Fremdmaterial gehörig zu erkennen, wenn das betreffende Element ausserhalb des charakteristischen Gebiets liegt. Gemäss einer ersten Alternative ist der Computer dazu eingerichtet, die Ausscheidung der Fremdmaterialien auszusetzen, bis ein statistisch repräsentatives charakteristisches Gebiet vorliegt und numerisch spezifiziert ist. Gemäss einer zweiten Alternative ist der Computer dazu eingerichtet, ein initiales charakteristisches Gebiet zu speichern und das gespeicherte charakteristische Gebiet laufend an eine Materialzusammensetzung des Textilfasergebildes anzupassen.

Gemäss einer Ausführungsform ist der Computer dazu eingerichtet, zusätzlich einen Auflösegrad des Textilfasergebildes, der angibt, in welchem Masse das Textilfasergebilde in einzelne Faserflocken aufgelöst ist, zu bestimmen und die Farbinformationen, einen Schwellenwert und/oder einen Toleranzbereich mit dem Auflösegrad zu korrigieren. Zu diesem Zweck kann das optische Sensorsystem einen Bildsensor zur Aufnahme mindestens eines Bilds des Textilfasergebildes beinhalten; der Computer kann dazu eingerichtet sein, aus dem mindestens einen Bild diejenige Bildfläche, auf die Fasermaterial abgebildet ist, durch Bildverarbeitung zu bestimmen, einen Massenfluss des Textilfasergebildes pro Zeiteinheit zu bestimmen und den Auflösegrad als skalare Grösse durch Bildung eines Quotienten aus der Bildfläche, auf die Fasermaterial abgebildet ist, einerseits und aus dem Massenfluss andererseits zu berechnen. Der Computer ist vorzugsweise dazu eingerichtet, den Auflösegrad gemäss der Formel

$$X = A_F / (A_T \cdot m)$$

zu bestimmen, worin $A_F$ diejenige Bildfläche, auf die Fasermaterial abgebildet wird, $A_T$ eine Gesamtfläche des Bildes und m einen Massenfluss des Textilfasergebildes pro Zeiteinheit bedeuten.

Die erfindungsgemässe optische Charakterisierung des Textilfasergebildes bietet mehrere Vorteile. So kann z. B. aus einer Änderung der numerischen Spezifikation der Häufigkeitsdichteverteilung auf eine Änderung einer Materialzusammensetzung des Textilfasergebildes geschlossen werden. Für eine Spinnerei ist es wichtig, eine solche Materialänderung frühzeitig festzustellen, denn sie kann auf eine fehlerhafte Zusammenstellung der Ballenvorlage hinweisen. Erfolgt die Feststellung in einer frühen Stufe des Spinnereiprozesses, z. B. an einem Faserreiniger in der Putzerei, so kann eine Herstellung von Garn aus dem falschen Textilfasermaterial durch eine entsprechende Korrektur noch rechtzeitig verhindert werden.

Ferner optimiert die Erfindung die Ausscheidung von Fremdmaterialien aus dem Textilfasergebilde. Aufgrund der erfindungsgemässen Charakterisierung kann eine an das aktuell verarbeitete Textilfasergebilde angepasste Grenze zwischen dem Grundmaterial des Textilfasergebildes und auszuscheidenden Fremdmaterialien gezogen und für die Ausscheidung der Fremdmaterialien verwendet werden. So wird sichergestellt, dass einerseits die Fremdmaterialien zuverlässig ausgeschieden werden, andererseits aber möglichst wenig Gutmaterial aus dem Textilfasergebilde verschwendet wird.

Insgesamt verhindert die Erfindung unerwünschte Qualitätsänderungen des textilen Fasergebildes und der daraus hergestellten textilen Endprodukte.

In der vorliegenden Schrift beziehen sich Begriffe wie «Textilfasermaterial» o. ä. immer auf das Grundmaterial, aus dem das Textilfasergebilde grossmehrheitlich besteht. Allfällig vorhanden Verunreinigungen durch einzelne Fremdmaterialien spielen für die vorliegende Erfindung keine unmittelbare Rolle und sind unberücksichtigt zu lassen, wenn hier von «Zusammensetzung des Textilfasermaterials», «Änderungen des Textilfasermaterials», «Materialänderungen» o. ä. die Rede ist.

AUFZÄHLUNG DER ZEICHNUNGEN

Nachfolgend wird eine Ausführungsform der Erfindung anhand der schematischen Zeichnungen detailliert erläutert.

Figur 1    zeigt eine Ausführungsform der erfindungsgemässen Vorrichtung.

Figur 2(a)    zeigt einen zweidimensionalen Farbraum mit einer Punktewolke und einem charakteristischen Gebiet.

Figur 2(b)    zeigt eine eindimensionale Häufigkeitsdichteverteilung der Punktewolke von Figur 2(a).

Figur 3    zeigt jeweils einen zweidimensionalen Farbraum mit einem charakteristischen Gebiet, wobei sich die charakteristischen Gebiete der Figuren 3(a) und 3(b) voneinander unterscheiden.

Figur 4    zeigt zeitliche Verläufe von Werten, die ein charakteristisches Gebiet numerisch spezifizieren.

Figur 5    zeigt einen dreidimensionalen RGB-Farbraum mit einem charakteristischen Gebiet.

## AUSFÜHRUNG DER ERFINDUNG

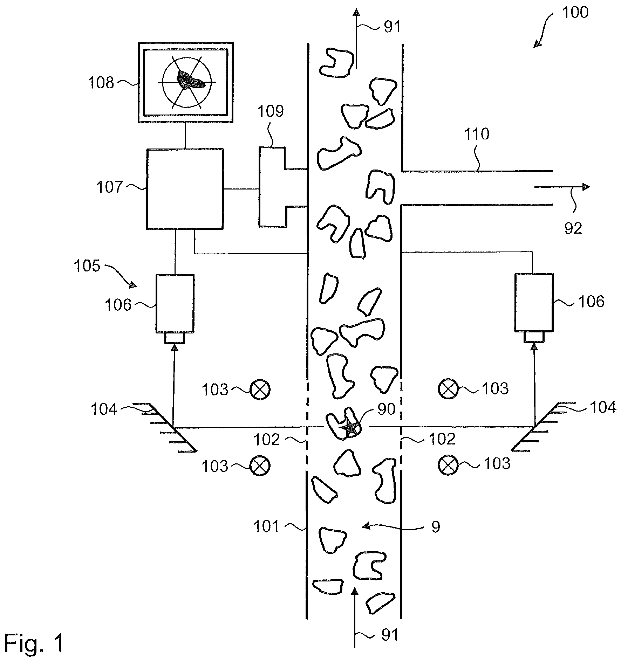

**Figur** 1 illustriert schematisch eine Ausführungsform der erfindungsgemässen Vorrichtung 100 anhand des Beispiels eines Faserreinigers in einer Putzerei. Die Vorrichtung 100 dient zur automatischen optischen Charakterisierung eines Textilfasergebildes 9, das im vorliegenden Beispiel ein Faserflockenstrom, bspw. ein Baumwollflockenstrom, ist. Sie beinhaltet einen pneumatischen Fasertransportkanal 101 zum pneumatischen Transport des Faserflockenstroms 9 in einem Luftstrom. Die Transportrichtung des Faserflockenstroms 9 und des Luftstroms ist in Figur 1 durch Pfeile 91 angedeutet.

Vier Lichtquellen 103, z. B. Leuchtstoffröhren oder LED-Arrays, sind in der Nähe von Fenstern 102 in einer Wand des Fasertransportkanals 101 angeordnet. Die Lichtquellen 103 beleuchten aus verschiedenen Richtungen den Faserflockenstrom 9 im Fasertransportkanal 101.

Ein optisches Sensorsystem 105 ist am Fasertransportkanal 101 angeordnet. Es erfasst Farbinformationen über den Faserflockenstrom 9. In der Ausführungsform von Figur 1 beinhaltet das Sensorsystem 105 zwei Kameras 106, die Bilder des Faserflockenstroms 9 durch die Fenster 102 aus zwei verschiedenen Richtungen aufnehmen. Das von den Lichtquellen 103 ausgesendete Licht wird nach einer Wechselwirkung mit dem Faserflockenstrom 9 mittels entsprechend gekippter Spiegel 104 zu den Kameras 106 abgelenkt. Die Kameras 106 sind z. B. mit CMOS- oder CCD-Fotosensoren ausgestattet, deren Pixel mit Mosaik-Farbfiltern in der Anordnung einer Bayer-Matrix überzogen sind. Die Ausgangssignale solcher Fotosensoren können innerhalb oder ausserhalb der Kameras 106 mittels an sich bekannter Algorithmen so interpoliert werden, dass jedem Pixel oder Bildsegment eine Farbe in einem Farbmodell zugeordnet wird.

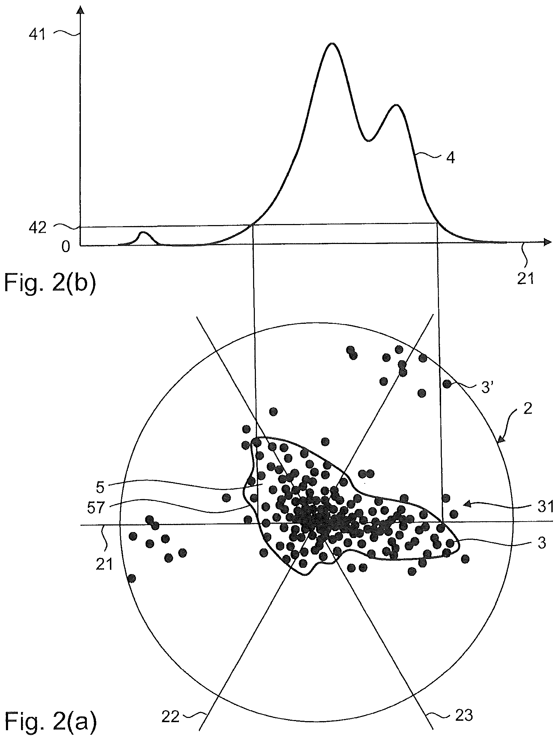

Die Kameras 106 sind mit einem Computer 107 zur automatischen Auswertung von Ausgangssignalen des optischen Sensorsystems 105 verbunden. Der Computer 107 ist dazu eingerichtet, die erfassten Farbinformationen in einem Farbraum als Punktewolke einzutragen, eine Häufigkeitsdichteverteilung der Punktewolke zu bestimmen und die Häufigkeitsdichteverteilung numerisch zu spezifizieren (siehe Figur 2 unten).

Der Computer 107 ist mit einer Ausgabeeinheit 108 verbunden. Ein vom Computer 107 ausgegebenes Signal kann eine Ausgabe eines Resultates der Charakterisierung, bspw. einer Grafik, auf der Ausgabeeinheit 108 bewirken. Die Ausgabeeinheit 108 kann z. B. ein Computerbildschirm oder ein Drucker sein. In einer Ausführungsform ist sie als Berührungsbildschirm ausgebildet und dient somit als Eingabe- und Ausgabeeinheit.

Eine Ausscheideeinheit 109 ist am Fasertransportkanal 101 stromabwärts vom Sensorsystem 105 (bezüglich der Transportrichtung 91) angeordnet. Die Ausscheideeinheit 109 dient zur selektiven Ausscheidung von Fremdmaterialien 90 aus dem Faserflockenstrom 9. Eine solche Ausscheideeinheit 109 ist an sich bekannt, z. B. aus der WO-2006/079426 A1. In einer bevorzugten Ausführungsform beinhaltet sie eine Mehrzahl von druckbeaufschlagten Luftdüsen, die einzeln oder gruppenweise durch ein vom Computer 107 ausgegebenes Signal ansteuerbar sind. Wenn das Sensorsystem 105 ein unzulässiges Fremdmaterial 90 im Faserflockenstrom 9 detektiert, so wird die betreffende Luftdüse der Ausscheideeinheit 109 durch das vom Computer 107 ausgegebene Signal veranlasst, Druckluft senkrecht zur Transportrichtung 91 auszublasen, wenn das Fremdmaterial 90 auf der Höhe der Ausscheideeinheit 109 angekommen ist. Dadurch wird das Fremdmaterial 90 in einen Ausscheidekanal 110, der aus dem Fasertransportkanal 101 in einer Ausscheiderichtung 92, die im Wesentlichen senkrecht zur Transportrichtung 91 liegt, ausgeblasen. Die unverschmutzten Faserflocken setzen dagegen ihren Weg mit dem Faserflockenstrom 9 fort, um weiter verarbeitet zu werden.

Die Ausscheideeinheit 109 kann vom Computer 107 und/oder direkt vom Sensorsystem 105 angesteuert werden. Im letzteren Fall kann jeder Kamera 106 ein Computer, bspw. in Form eines Mikroprozessors, zugeordnet sein, und die Kameras 106 können direkt mit der Ausscheideeinheit 109 verbunden sein und ihre Signale an diese ausgeben. Solche direkten Verbindungen sind in Figur 1 der Einfachheit halber nicht eingezeichnet. In einer weiteren Alternative wird die Ausscheideeinheit 109 durch einen Computer angesteuert, welcher der Ausscheideeinheit 109 selbst zugeordnet ist.

In Figur 2(a) ist schematisch ein zweidimensionaler Farbraum 2 dargestellt. Dieser Farbraum 2 gibt Informationen über den Farbton und die Farbsättigung, nicht aber über die Helligkeit wieder. Es kann sich z. B. um eine horizontale Schnittebene durch den bekannten HSV-Farbraum handeln; in Polarkoordinaten entspricht der Winkel dem Farbton (H) und der Radius der Farbsättigung (S). Statt eines solchen wahrnehmungsorientierten Farbmodells wird jedoch für die vorliegende Erfindung ein technisch-physikalisches Farbmodell bevorzugt. Ein vorteilhafter Farbraum 2 ist z. B. der II I2I3-Farbraum ohne die Helligkeitskomponente (I1).

Erfindungsgemäss wird eine Vielzahl von Farbinformationen über verschiedene Stellen des Textilfasergebildes 9 vom optischen Sensorsystem 105 erfasst, vorzugsweise stichprobenartig. Eine Stichprobe kann Farbinformationen aus mehreren Stellen eines einzigen Bildes und/oder Farbinformationen aus verschiedenen Bildern umfassen. Vorzugsweise besteht sie aus einer Vielzahl von Farbinformationen aus jeweils mehreren Pixeln mehrerer digitaler Bilder. Dabei braucht es sich nicht notwendigerweise um unmittelbar nacheinander aufgenommene Bilder zu handeln; bspw. kann ein Bild pro Sekunde genügen, während die Bildfrequenz viel höher ist. Die Stichprobe sollte eine derart grosse Anzahl Elemente enthalten, dass sie für das Textilfasergebilde 9 statistisch repräsentativ ist. Bei Kenntnis der Erfindung ist der Fachmann in der Lage, den erforderlichen Mindestumfang der Stichprobe zu bestimmen. Die entsprechenden Grundlagen für eine theoretische Betrachtung sind z. B. im Buch «Statistische Methoden bei textilen Untersuchungen» von Graf, Henning und Wilrich, 2. Aufl., Springer-Verlag, 1974, dargelegt.

Die erfassten Farbinformationen werden vom Sensorsystem 105 an den Computer 107 gesendet, von diesem empfangen und gespeichert. Die gespeicherten Farbinformationen werden vom Computer 107 in einem Farbraum, z. B. im zweidimensionalen Farbraum 2 von Figur 2(a), als durch Punkte dargestellte Farbereignisse 3 in einer Punktewolke 31 eingetragen. Die Darstellung der Punktewolke 31 kann aufgrund des vom Computer 107 ausgegebenen Signals auf der Ausgabeeinheit 108 als Grafik an eine Bedienperson ausgegeben werden, was aber fakultativ ist; alternativ kann die Eintragung der Farbinformationen rein virtuell in dem Computer 107 erfolgen.

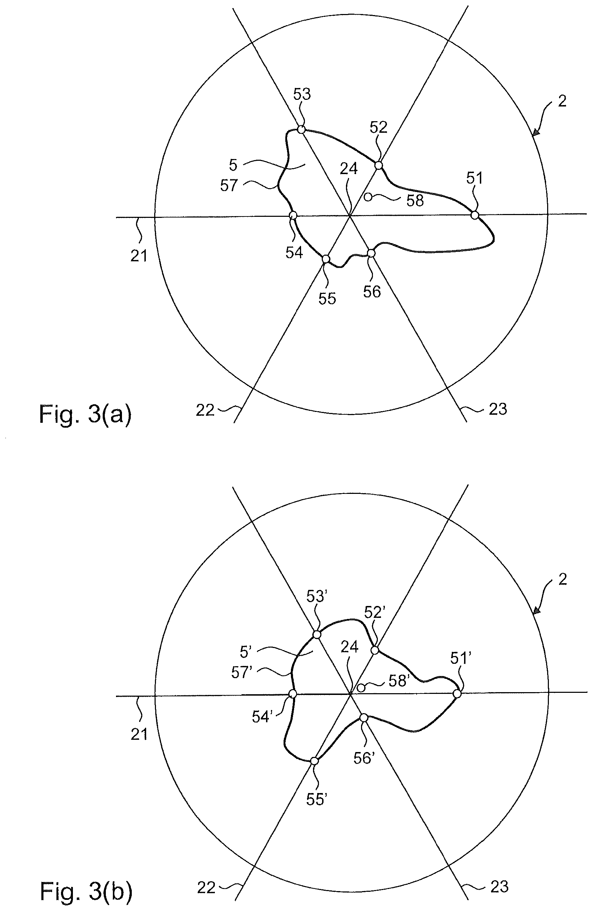

Anschliessend wird vom Computer 107 eine Häufigkeitsdichteverteilung der Punktewolke 31 bestimmt. Da es sich bei den Farbinformationen um diskrete Messwerte handelt, wird die so bestimmte Häufigkeitsdichteverteilung Stufen aufweisen. Sie kann, braucht aber nicht, durch eine stetige Funktion rechnerisch angenähert werden; entsprechende Rechenverfahren sind bekannt. Eine beispielhafte, als stetig angenäherte eindimensionale Häufigkeitsdichteverteilung 4 entlang einer Achse 21 des Farbraums 2 von Figur 2(a) ist in **Figur 2(b)** schematisch eingezeichnet, wobei die vertikale Achse 41 die Häufigkeitsdichte angibt. Die Häufigkeitsdichteverteilung 4 wird nicht nur in einer Dimension (wie der Einfachheit halber in Figur 2(b) dargestellt), sondern im ganzen zweidimensionalen Farbraum 2 von Figur 2(a) bestimmt. Für annähernd weisse Baumwolle ist die Häufigkeitsdichte in einer Umgebung eines im Koordinatenursprung des Farbraums 2 liegenden Grau- bzw. Weisspunktes 24 (siehe Figur 3) am grössten, doch braucht das Maximum nicht auf dem Graupunkt 24 zu liegen, denn jede Baumwolle hat ihre charakteristische Färbung. Mit der automatischen Charakterisierung dieser Färbung befasst sich die vorliegende Erfindung.

Erfindungsgemäss wird die Häufigkeitsdichteverteilung 4 vom Computer 107 numerisch spezifiziert. Der Fachmann weiss dank seiner Grundausbildung in Mathematik, wie eine Kurve bzw. eine Fläche numerisch spezifiziert werden kann: z. B. durch ihre Extremwerte, Sattelpunkte, Gradienten an bestimmten Stellen in bestimmte Richtungen, Annäherung durch eine geeignete Fitfunktion etc. Nachfolgend wird ohne Einschränkung der Allgemeinheit eine bestimmte Art der numerischen Spezifikation der Häufigkeitsdichteverteilung 4 beispielhaft diskutiert, die sich für Textilfasergebilde 9 besonders gut eignet.

Aufgrund der Häufigkeitsdichteverteilung 4 wird vom Computer 107 ein charakteristisches Gebiet 5 in dem Farbraum 2 definiert. Das charakteristische Gebiet 5 braucht nicht zusammenhängend zu sein. Im einfachsten Fall wird das charakteristische Gebiet 5 durch eine Grenzlinie 57 mit konstanter Häufigkeitsdichte 42 begrenzt. Die entsprechende Häufigkeitsdichte 42 wird so gewählt, dass ein grosser Teil, bspw. 95 % oder 99 %, der Punktewolke 31 innerhalb des charakteristischen Gebiets 5 liegt. Die Farbereignisse 3' ausserhalb des charakteristischen Gebiets 5 sind dann Ausreisser, die nicht als charakteristisch für das Textilfasergebilde 9 betrachtet werden und deshalb unberücksichtigt bleiben. Solche Ausreisser 3' können z. B. durch Fehlmessungen zustande kommen oder stellen Fremdmaterialien 90 dar, die sich unerwünschterweise im Textilfasergebilde 9 befinden.

Die Grenzlinie 57 braucht nicht notwendigerweise durch eine durch eine konstante Häufigkeitsdichte 42 definiert zu sein. Sie kann z. B. festgelegt werden, indem zu einer Linie mit konstanter Häufigkeitsdichte 42 ein konstanter Sicherheitsabstand eingehalten wird, so dass sie im Farbraum 2 weiter aussen liegt. Alternativ kann sie z. B. durch Streckung der zu einer Linie mit konstanter Häufigkeitsdichte 42 gehörenden Radien mit einem konstanten Streckfaktor hervorgehen. Alle derartigen Möglichkeiten zur Definition des charakteristischen Gebiets 5 müssen jedoch die Häufigkeitsdichteverteilung 4 als Grundlage verwenden.

Das charakteristische Gebiet 5 wird seinerseits vom Computer 107 numerisch spezifiziert, was in **Figur 3(a)** beispielhaft illustriert ist. Die numerische Spezifikation kann z. B. die folgenden Eigenschaften des charakteristischen Gebiets 5 berücksichtigen:

- eine Länge, einen Flächeninhalt bzw. ein Volumen des charakteristischen Gebiets 5, je nachdem, ob das Gebiet 5 ein-, zwei- oder dreidimensional ist,
- eine geometrische Form des charakteristischen Gebiets 5, in zwei Dimensionen z. B. eine Lage eines Flächenschwerpunktes 58 oder eines Konturenschwerpunktes des Gebiets 5, und/oder
- einen Verlauf einer Grenzlinie 57 bzw. einer Grenzfläche, die das charakteristische Gebiet 5 begrenzt. Dabei kann die numerische Spezifikation anhand von Stützpunkten erfolgen. In Figur 3(a) sind z. B. sechs Stützpunkte 51-56 eingezeichnet, die paarweise auf Achsen 21-23 liegen, die sich im Koordinatenursprung (Graupunkt 24) schneiden und einen Winkelabstand von 60° zueinander aufweisen. In diesem Beispiel ist jeder Stützpunkt 51-56 durch seine radiale Entfernung vom Koordinatenursprung eindeutig bestimmt. Zur numerischen Spezifizierung können die zu den Stützpunkten 51-56 gehörigen Entfernungswerte einzeln angegeben oder zu einem einzigen Wert miteinander mathematisch verknüpft werden.

Nachfolgend werden zwei bevorzugte Anwendungsbeispiele für das erfindungsgemässe Verfahren und die erfindungsgemässe Vorrichtung 100 beschrieben.

Ein erstes Anwendungsbeispiel hat zum Ziel, eine zeitliche Veränderung des Textilfasermaterials bzw. seiner Zusammensetzung festzustellen und eine entsprechende Handlung automatisch auszulösen, bspw. ein Warnsignal auszugeben. Dank des Warnsignals kann z. B. eine fehlerhafte Zusammenstellung der Ballenvorlage festgestellt werden.

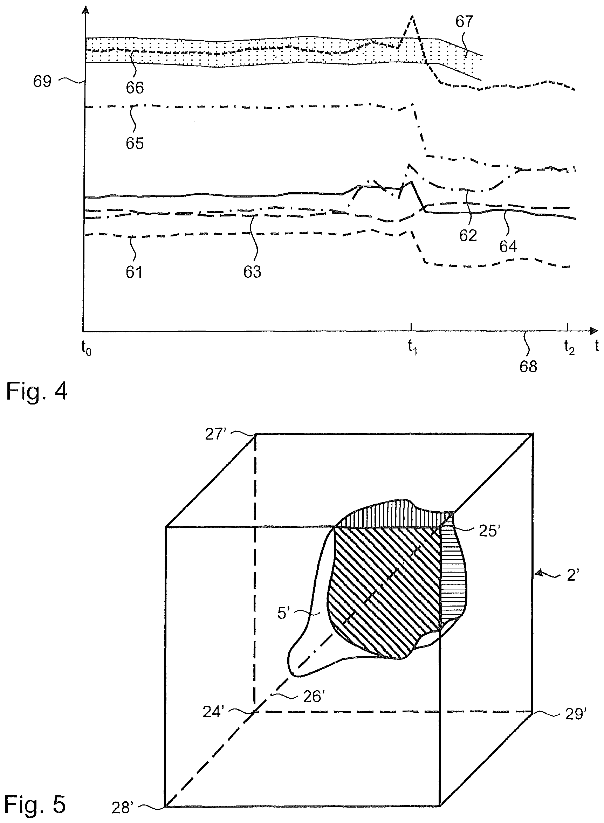

**Figur 4** illustriert eine Ausführungsform des ersten Anwendungsbeispiels. Darin sind Werte der Radialkomponente der sechs Stützpunkte 51-56 von Figur 3 entlang einer vertikalen Achse 69 gegenüber der Zeit t entlang einer horizontalen Zeitachsechse 68 als Kurven 61-66 aufgetragen. Jede Kurve 61-66 stellt den Zeitverlauf einer auf der entsprechenden Achse 21-23 (Figur 3) liegenden Farbkomponente dar, z. B. Grün 61, Cyan 62, Blau 63, Rot 64, Magenta 65 und Gelb 66. Im Normalfall genügt es, die Werte periodisch in grösseren Zeitabständen zu erfassen, z. B. alle 15 Minuten, so dass das Diagramm von Figur 4 einen Zeitraum von mehreren Stunden abdeckt.

Im Beispiel von Figur 4 verändern sich die Werte in den ersten Stunden nur unwesentlich und liegen somit innerhalb eines Toleranzbereichs. Zu einem bestimmten Zeitpunkt $t_1$ beginnen sie sich jedoch merklich zu ändern. Dies kann ein Hinweis auf eine fehlerhafte Zusammenstellung der Ballenvorlage sein. Die Materialänderung kann anhand eines einzigen Wertes in Figur 4 und/oder anhand einer Verknüpfung der Werte vom Computer 107 festgestellt werden. Zu diesem Zweck können z. B. Toleranzbereiche vorgegeben werden, bei deren Überschreitung der Computer 107 ein Signal ausgibt. Dabei können die aktuellen Werte oder laufende Mittelwerte aus mehreren nacheinander erfassten Werten berücksichtigt werden. In Figur 4 ist für die Gelb-Kurve 66 ein Toleranzbereich 67 in Form eines die Kurve 66 anfangs umgebenden Bandes angedeutet. Der Toleranzbereich 67 wird vorzugsweise einem laufenden Mittelwert der Kurve 66 nachgeführt, um eine Drift, die nicht durch eine Materialänderung verursacht wird, auszugleichen. Um den Zeitpunkt $t_1$ herum verlässt die Kurve 66 den Toleranzbereich 67, was die Ausgabe des Signals zur Folge hat.

Das vom Computer 107 ausgegebene Signal kann z. B. mindestens eine der folgenden Handlungen automatisch auslösen: Ausgabe einer Grafik an eine Bedienperson, Ausgabe einer Warnung an eine Bedienperson, Ausgabe einer Handlungsempfehlung an eine Bedienperson, Abstellen mindestens einer Verarbeitungsmaschine, Änderung einer Einstellung an mindestens einer Verarbeitungsmaschine.

**Figur 3** veranschaulicht, wie sich bei einem Zeitverlauf gemäss Figur 4 das charakteristische Gebiet 5 verändern kann. In **Figur 3(a)** ist beispielhaft ein charakteristisches Gebiet 5 zu einem Anfangszeitpunkt $t_0$, in **Figur 3(b)** ein verändertes charakteristisches Gebiet 5' zu einem späteren Zeitpunkt $t_2 > t_1 > t_0$ dargestellt. Die Form des charakteristischen Gebiets 5 hat sich im Zeitintervall $[t_0, t_2]$ sichtlich verändert, was durch die oben beschriebene numerische Spezifikation quantitativ erfasst wird. Das charakteristische Gebiet 5' zum späteren Zeitpunkt $t_2$ hat andere Stützpunkte 51'-56' (vgl. auch Figur 4), eine andere Grenzlinie 57', einen anderen Flächenschwerpunkt 58', einen anderen Flächeninhalt etc. als das charakteristische Gebiet 5 zum Anfangszeitpunkt $t_0$.

Ein zweites Anwendungsbeispiel befasst sich mit der Ausscheidung von Fremdmaterialien 90 aus dem Textilfasergebilde 9 (vgl. Figur 1). Das charakteristische Gebiet 5 mit seinen Farbereignissen 3 ist charakteristisch für das Textilfasergebilde 9, während alle Farbereignisse 3' ausserhalb des Gebiets 5 als Fremdmaterialien 90 betrachtet werden, die ausgeschieden werden sollen. Um Fehlausscheidungen zu vermeiden, soll das charakteristische Gebiet 5 an das jeweils verarbeitete Textilfasergebilde 9 angepasst werden.

Gemäss einer ersten Alternative kann während einer Lernphase zu Beginn der Verarbeitung eines neuen Loses von Textilfasermaterial die Fremdmaterialausscheidung ausgesetzt werden. Die Farbinformationen über das Textilfasergebilde 9 werden so lange erfasst und gespeichert, bis ein statistisch repräsentatives charakteristisches Gebiet 5 vorliegt und numerisch spezifiziert ist. Sobald dies der Fall ist, wird die Fremdmaterialausscheidung aktiviert. Sie bewirkt, dass alle Farbereignisse 3' ausserhalb des charakteristischen Gebiets 5 aus dem Textilfasergebilde 9 als Fremdmaterialien 90 ausgeschieden werden. Die Ausscheidung erfolgt mittels der Ausscheideeinheit 109, die durch ein vom Computer 107 ausgegebenes Signal gesteuert wird. Diese erste Alternative hat den Nachteil, dass während der Lernphase das Textilfasergebilde 9 ungereinigt in die nachfolgenden Prozessschritte gelangt. Die Lernphase ist aber verglichen mit der gesamten Verarbeitungszeit des Loses so kurz, dass die der Erfindung zu verdankende verbesserte Fremdmaterialausscheidung den Nachteil bei Weitem überwiegt.

Gemäss einer zweiten Alternative werden von Anfang an Fremdmaterialien 90 ausgeschieden. Um dies zu ermöglichen, wird vor Beginn der Verarbeitung ein initiales charakteristisches Gebiet 5 vorgegeben. Dieses kann z. B. ein für ein früher verarbeitetes Textilfasermaterial ermitteltes charakteristisches Gebiet sein, durch eine einfache Farbmessung am zu verarbeitenden Textilfasermaterial ermittelt oder aufgrund von theoretischen Überlegungen entworfen werden. Während der Verarbeitung kann dann das initiale charakteristische Gebiet 5 vom Computer 107 laufend immer besser an das aktuelle Textilfasermaterial angepasst werden. Die Ausscheidung erfolgt wiederum mittels der Ausscheideeinheit 109, die durch ein vom Computer 107 ausgegebenes Signal gesteuert wird.

In beiden Alternativen kann die zu erwartende oder aktuelle Ausscheiderate, d. h. die Anzahl Ausscheidungen pro Zeiteinheit oder pro Masseneinheit des Textilfasergebildes 9, vom Computer 107 auf der Ausgabeeinheit 108 (Figur 1) an eine Bedienperson ausgegeben werden. Der Bedienperson kann die Möglichkeit gegeben werden, über die Eingabeeinheit 108 die ausgegebene Ausscheiderate zu bestätigen oder zu ändern. Eine Änderung der Ausscheiderate kann in einer Senkung oder in einer Erhöhung derselben bestehen. Im ersteren Fall wird das charakteristische Gebiet 5 (Figur 2(a)) vergrössert, im letzteren Fall verkleinert. Dank der bereits vorliegenden numerischen Spezifikation der Häufigkeitsdichteverteilung 4 (Figur 2(b)) kann eine solche gezielte Änderung schnell und einfach vom Computer 107 automatisch durchgeführt werden.

**Figur 5** verdeutlicht, dass im erfindungsgemässen Verfahren nicht nur zweidimensionale, sondern z. B. auch dreidimensionale Farbräume verwendet werden können. Sie zeigt den bekannten dreidimensionalen RGB-Farbraum 2' mit einem Schwarzpunkt 24', einem Weisspunkt 25', einem Rotpunkt 27', einem Grünpunkt 28' und einem Blaupunkt 29'. In dem RGB-Farbraum 2' ist ein charakteristischen Gebiet 5' gemäss dem erfindungsgemässen Verfahren schematisch eingezeichnet. Das in diesem Fall dreidimensionale charakteristische Gebiet 5' basiert auf einer Häufigkeitsdichteverteilung einer dreidimensionalen Punktewolke, die jedoch in Figur 5 der Übersichtlichkeit halber nicht eingezeichnet ist. Für annähernd weisse Baumwolle ist die Häufigkeitsdichte in einer Umgebung einer Grauwertachse 26' (Raumdiagonale, die den Schwarzpunkt 24' mit dem Weisspunkt 25' verbindet) am grössten.

Während die zweidimensionale Farbinformation gemäss den Figuren 2(a) und 3 von der Helligkeit zumindest im Idealfall nicht beeinflusst wird, enthält die dreidimensionale Farbinformation gemäss Figur 5 die Helligkeit sehr wohl. Nun wird aber in einer Vorrichtung 100 gemäss Figur 1 die gemessene Helligkeit durch die Auflösung des Textilfasergebildes 9 in einzelne Faserflocken beeinflusst: Je besser das Textilfasergebilde 9 in einzelne Faserflocken aufgelöst ist, umso grösser ist die Helligkeit, und umgekehrt. Deshalb kann es vorteilhaft sein, insbesondere im Farbraum 2' von Figur 5 die Farbinformationen mit einem Auflösegrad, der angibt, in welchem Masse das Textilfasergebilde 9 in einzelne Faserflocken aufgelöst ist, zu korrigieren.

Der Auflösegrad und seine Bestimmung werden in der WO-2017/ 117688 A1 ausführlich diskutiert. Vorzugsweise wird der Auflösegrad als skalare Grösse durch Bildung eines Quotienten aus der Bildfläche $A_F$ eines von der Kamera 106 aufgenommenen Bildes, auf die Fasermaterial abgebildet wird, einerseits und aus dem Massenfluss m des Faserflockenstroms 9 andererseits berechnet. Eine beispielhafte Definition des Auflösegrades X lautet:

$$X = A_F / \left( A_T \cdot m \right) \ ,$$

worin $A_F$ diejenige Bildfläche, $A_T$ eine Gesamtfläche des Bildes und m einen Massenfluss des Faserflockenstroms 9 pro Zeiteinheit bedeuten. Kleinere Werte von X geben in diesem Beispiel eine niedrigere, schlechtere Auflösung des Faserflockenstroms 9, grössere Werte eine höhere, bessere Auflösung an.

Die Korrektur der Farbinformation mit dem Auflösegrad X kann mit Hilfe einer theoretischen Formel oder von empirisch bestimmten Korrekturwerten, die z. B. in einer Tabelle gespeichert sein können, erfolgen. Statt oder zusätzlich zu den Farbinformationen kann ein Schwellenwert und/oder ein Toleranzbereich mit dem Auflösegrad X korrigiert werden. Die Korrektur mit dem Auflösegrad X kann unter Umständen nicht nur in dreidimensionalen Farbräumen 2', sondern auch in zweidimensionalen Farbräumen 2 sinnvoll sein. Die Korrektur erfolgt durch den Computer 107.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

In der obigen Beschreibung wird die Erfindung anhand von Beispielen mit zweidimensionalen Farbräumen 2 (Figuren 2(a) und 3) und dreidimensionalen Farbräumen 2' (Figur 5) diskutiert. Der Fachmann ist in der Lage, die vorliegende Erfindung auch an einen eindimensionalen Farbraum anzupassen.

Ebenso ist der Fachmann in der Lage, die Erfindung von den hier diskutierten Farbräumen 2, 2' im sichtbaren Bereich des elektromagnetischen Spektrums auf Farbräume zu verallgemeinern, welche die mit dem sichtbaren Bereich benachbarten Spektralbereiche Ultraviolett und Infrarot oder Teile davon umfassen.

In der obigen Beschreibung wir die Erfindung anhand des Beispiels eines Faserreinigers in einer Putzerei diskutiert, wobei das Textilfasergebilde 9 in Form eines Faserflockenstroms pneumatisch durch die Vorrichtung 100 (Figur 1) transportiert wird. Die Erfindung ist jedoch nicht auf diese Anwendung beschränkt. Sie kann ebenso z. B. in der Spinnereivorbereitung, wo das Textilfasergebilde in Form eines Faserbandes vorliegt, oder in der Spinnerei/Spulerei, wo das Textilfasergebilde in Form von Garn vorliegt, eingesetzt werden.

BEZUGSZEICHENLISTE

| | |
|---|---|
| 100 | erfindungsgemässe Vorrichtung |
| 101 | Fasertransportkanal |
| 102 | Fenster in Wand des Fasertransportkanals |
| 103 | Lichtquellen |
| 104 | Spiegel |
| 105 | Sensorsystem |
| 106 | Kameras |
| 107 | Computer |
| 108 | Ausgabe- und Eingabeeinheit |
| 109 | Ausscheideeinheit |
| 110 | Ausscheidekanal |

| | |
|---|---|
| 2, 2' | Farbraum |
| 21-23 | Achsen des Farbraums |
| 24 | Graupunkt |
| 24' | Schwarzpunkt |
| 25' | Weisspunkt |
| 26' | Grauwertachse |
| 27' | Rotpunkt |
| 28' | Grünpunkt |
| 29' | Blaupunkt |

| | |
|---|---|
| 3 | Farbereignisse |
| 3' | Ausreisser |
| 31 | Punktewolke |

| | |
|---|---|
| 4 | Häufigkeitsdichteverteilung |
| 41 | Häufigkeitsdichtenachse |

| | |
|---|---|
| 5, 5' | charakteristisches Gebiet |
| 51-56, 51'-56' | Stützpunkte |
| 57, 57' | Grenzlinie |
| 58, 58' | Flächenschwerpunkt |

| | |
|---|---|
| 61-66 | Kurven, die Zeitverläufe von Farbkomponenten darstellen |
| 67 | Toleranzbereich |
| 68 | Zeitachse |
| 69 | vertikale Diagrammachse |

| | |
|---|---|
| 9 | Faserflockenstrom |
| 90 | Fremdmaterial |
| 91 | Transportrichtung des Faserflockenstroms |
| 92 | Ausscheiderichtung |

**Patentansprüche**

1. Computerimplementiertes Verfahren zur optischen Charakterisierung eines Textilfasergebildes (9), wobei

   eine Vielzahl von Farbinformationen über verschiedene Stellen des Textilfasergebildes (9) von einem optischen Sensorsystem (105) erfasst wird, die erfassten Farbinformationen vom optischen Sensorsystem (105) an einen mit dem optischen Sensorsystem verbundenen Computer (107) gesendet, von diesem empfangen und gespeichert werden,
   die gespeicherten Farbinformationen vom Computer (107) in einem Farbraum (2, 2') als Punktewolke (31) eingetragen werden,
   **dadurch gekennzeichnet, dass**
   eine Häufigkeitsdichteverteilung (4) der Punktewolke (31) vom Computer (107) bestimmt wird,
   die Häufigkeitsdichteverteilung (4) vom Computer (107) numerisch spezifiziert wird und
   ein von der numerischen Spezifikation abhängiges Signal vom Computer (107) ausgegeben wird.

2. Verfahren nach Anspruch 1, wobei der Farbraum (2) ein ein- oder zweidimensionaler Farbraum ist, in dem Information über eine Helligkeit unberücksichtigt bleibt.

3. Verfahren nach Anspruch 1 oder 2, wobei vom Computer (107) aufgrund der Häufigkeitsdichteverteilung (4) ein charakteristisches Gebiet (5, 5') in dem Farbraum (2, 2') definiert wird und zur numerischen Spezifikation der Häufigkeitsdichteverteilung (4) das charakteristische Gebiet (5, 5') numerisch spezifiziert wird.

4. Verfahren nach Anspruch 3, wobei vom Computer (107) zur numerischen Spezifikation des charakteristischen Gebiets (5, 5') eine Länge, ein Flächeninhalt bzw. ein Volumen des charakteristischen Gebiets (5, 5'), eine geometrische Form des charakteristischen Gebiets (5, 5') und/oder ein Verlauf einer Grenzlinie (57) bzw. einer Grenzfläche, die das charakteristische Gebiet (5, 5') begrenzt, verwendet wird.

5. Verfahren nach einem der vorangehenden Ansprüche, wobei vom Computer (107) aus einer Änderung der numerischen Spezifikation der Häufigkeitsdichteverteilung (4) auf eine Änderung einer Materialzusammensetzung des Textilfasergebildes (9) geschlossen wird und das vom Computer (107) ausgegebene Signal von der Änderung der Materialzusammensetzung abhängig ist.

6. Verfahren nach Anspruch 5, wobei das vom Computer (107) ausgegebene Signal verwendet wird, um mindestens eine der folgenden Handlungen auszulösen: Ausgabe einer Grafik an eine Bedienperson, Ausgabe einer Warnung an eine Bedienperson, Ausgabe einer Handlungsempfehlung an eine Bedienperson, Abstellen mindestens einer Verarbeitungsmaschine, Änderung einer Einstellung an mindestens einer Verarbeitungsmaschine.

**7.** Verfahren nach einem der vorangehenden Ansprüche, wobei vom Computer (107) aufgrund der numerischen Spezifikation der Häufigkeitsdichteverteilung (4) ein Fremdmaterial (90) im Textilfasergebilde (9) erkannt wird und das vom Computer ausgegebene Signal eine Ausscheidung des erkannten Fremdmaterials (90) aus dem Textilfasergebilde (9) steuert.

**8.** Verfahren nach den Ansprüchen 3 oder 4 einerseits und 7 andererseits, wobei vom Computer (107) ein Element der Punktewolke (31) als zu einem Fremdmaterial (90) gehörig erkannt wird, wenn das betreffende Element ausserhalb des charakteristischen Gebiets (5, 5') liegt.

**9.** Verfahren nach einem der vorangehenden Ansprüche, wobei vom Computer (107) die Farbinformationen, ein Schwellenwert und/oder ein Toleranzbereich mit einem Auflösegrad, der angibt, in welchem Masse das Textilfasergebilde (9) in einzelne Faserflocken aufgelöst ist, korrigiert werden.

**10.** Verfahren nach Anspruch 9, wobei vom optischen Sensorsystem (105) mindestens ein Bild des Textilfasergebildes (9) aufgenommen und vom Computer aus dem mindestens einen Bild diejenige Bildfläche, auf die Fasermaterial abgebildet ist, durch Bildverarbeitung bestimmt wird,

vom Computer (107) ein Massenfluss des Textilfasergebildes (9) pro Zeiteinheit bestimmt wird und
vom Computer (107) der Auflösegrad als skalare Grösse durch Bildung eines Quotienten aus der Bildfläche, auf die Fasermaterial abgebildet ist, einerseits und aus dem Massenfluss andererseits berechnet wird.

**11.** Verfahren nach Anspruch 9 oder 10, wobei vom Computer (107) der Auflösegrad gemäss der Formel

$$X = A_F \big/ \big( A_T \cdot m \big)$$

bestimmt wird, worin $A_F$ diejenige Bildfläche, auf die Fasermaterial abgebildet wird, $A_T$ eine Gesamtfläche des Bildes und m einen Massenfluss des Textilfasergebildes (9) pro Zeiteinheit bedeuten.

**12.** Vorrichtung (100) zur optischen Charakterisierung eines Textilfasergebildes (9), mit einem optischen Sensorsystem (105) zur Erfassung einer Vielzahl von Farbinformationen über verschiedene Stellen des Textilfasergebildes (9) und einem mit dem optischen Sensorsystem (105) verbundenen Computer (107), wobei das optische Sensorsystem (105) Sendemittel zum Senden der erfassten Farbinformationen an den Computer (107) aufweist,

der Computer (107) Empfangsmittel zum Empfangen der vom optischen Sensorsystem (105) gesendeten Farbinformationen aufweist und der Computer (107) dazu eingerichtet ist,

die erfassten Farbinformationen in einem Farbraum (2, 2') als Punktewolke (31) einzutragen,
eine Häufigkeitsdichteverteilung (4) der Punktewolke (31) zu bestimmen,
die Häufigkeitsdichteverteilung (4) numerisch zu spezifizieren und
ein von der numerischen Spezifikation abhängiges Signal auszugeben.

**13.** Vorrichtung (100) nach Anspruch 12, wobei der Computer (107) dazu eingerichtet ist, aufgrund der Häufigkeitsdichteverteilung (4) ein charakteristisches Gebiet (5, 5') in dem Farbraum (2, 2') zu definieren und zur numerischen Spezifikation der Häufigkeitsdichteverteilung (4) das charakteristische Gebiet (5, 5') numerisch zu spezifizieren.

**14.** Vorrichtung (100) nach Anspruch 12 oder 13, wobei der Computer (107) dazu eingerichtet ist, aus einer Änderung der numerischen Spezifikation der Häufigkeitsdichteverteilung (4) auf eine Änderung einer Materialzusammensetzung des Textilfasergebildes (9) zu schliessen und das vom Computer (107) ausgegebene Signal von der Änderung der Materialzusammensetzung abhängig ist.

**15.** Vorrichtung (100) nach Anspruch 14, wobei der Computer (107) so eingerichtet ist, dass das von ihm ausgegebene Signal geeignet ist, mindestens eine der folgenden Handlungen auszulösen: Ausgabe einer Grafik an eine Bedienperson, Ausgabe einer Warnung an eine Bedienperson, Ausgabe einer Handlungsempfehlung an eine Bedienperson, Abstellen mindestens einer Verarbeitungsmaschine, Änderung einer Einstellung an mindestens einer Verarbeitungsmaschine.

**16.** Vorrichtung (100) nach Anspruch 12 oder 13, wobei

die Vorrichtung (100) zusätzlich eine mit dem Computer (107) verbundene Ausscheideeinheit (109) beinhaltet und
der Computer (107) dazu eingerichtet ist, aufgrund der numerischen Spezifikation der Häufigkeitsdichteverteilung (4) ein Fremdmaterial (90) im Textilfasergebilde (9) zu erkennen und bei Erkennung des Fremdmaterials (90) mittels des von ihm ausgegebenen Signals eine Ausscheidung des erkannten Fremdmaterials (90) durch die Ausscheideeinheit (109) zu steuern.

**17.** Vorrichtung (100) nach den Ansprüchen 13 und 16, wobei der Computer (107) dazu eingerichtet ist, ein Element der Punktewolke (31) als zu einem Fremdmaterial (90) gehörig zu erkennen, wenn das betreffende Element ausserhalb des charakteristischen Gebiets (5, 5') liegt.

## Claims

**1.** Computer-implemented method for the optical characterization of a textile fiber structure (9), wherein

> a plurality of pieces of information on color is detected by an optical sensor system (105) at different locations of the textile fiber structure (9),
> the captured color information is transmitted from the optical sensor system (105) to a computer (107) connected to the optical sensor system, where it is received and stored by the computer,
> the stored color information is entered by the computer (107) in a color space (2, 2') in the form a scatter plot (31),
> **characterized in that**
> a frequency density distribution (4) of the scatter plot (31) is determined by the computer (107),
> the frequency density distribution (4) is specified numerically by the computer (107), and
> a signal dependent on the numerical specification is output by the computer (107).

**2.** Method according to claim 1, wherein the color space (2) is a one- or two-dimensional color space in which information about a brightness is not taken into account.

**3.** Method according to claim 1 or 2, wherein a characteristic region (5, 5') in the color space (2, 2') is defined by the computer (107) on the basis of the frequency density distribution (4) and the characteristic region (5, 5') is specified numerically for the numerical specification of the frequency density distribution (4).

**4.** Method according to claim 3, wherein a length, an area or a volume of the characteristic region (5, 5'), a geometric shape of the characteristic region (5, 5') and/or a course of a boundary line (57) or a boundary surface which delimits the characteristic region (5, 5') is used by the computer (107) for the numerical specification of the characteristic region (5, 5').

**5.** Method according to one of the preceding claims, wherein a change in a material composition of the textile fiber structure (9) is inferred by the computer (107) from a change in the numerical specification of the frequency density distribution (4), and the signal output by the computer (107) is dependent on the change in the material composition.

**6.** Method according to claim 5, wherein the signal output by the computer (107) is used to trigger at least one of the following actions: outputting a graphic to an operator, outputting a warning to an operator, outputting a recommended action to an operator, shutting down at least one processing machine, changing a setting on at least one processing machine.

**7.** Method according to one of the preceding claims, wherein the computer (107) recognizes a foreign material (90) in the textile fiber structure (9) on the basis of the numerical specification of the frequency density distribution (4), and the signal output by the computer controls a separation of the recognized foreign material (90) from the textile fiber structure (9).

**8.** Method according to the claims 3 or 4 on the one hand, and 7 on the other hand, wherein an element of the scatter plot (31) is recognized by the computer (107) as belonging to a foreign material (90) if the element in question lies outside the characteristic region (5, 5').

**9.** Method according to one of the preceding claims, wherein the computer (107) corrects the color information, a threshold value and/or a tolerance range with a degree of dissolution which indicates the extent to which the textile fiber structure (9) is dissolved into individual fiber flocks.

**10.** Method according to claim 9, wherein at least one image of the textile fiber structure (9) is recorded by the optical sensor system (105) and the image area onto which fiber material is imaged is determined by the computer from the at least one image by image processing,

> the computer (107) determines a mass flow of the textile fiber structure (9) per time unit, and
> the computer (107) calculates the degree of dissolution as a scalar quantity by forming a quotient from the image area onto which the fiber material is imaged on the one hand and from the mass flow on the other hand.

**11.** Method according to claim 9 or 10, wherein the computer (107) determines the degree of dissolution according to the formula

$$X = A_F / \left( A_T \cdot m \right) ,$$

wherein $A_F$ is the image area onto which fiber material is imaged, $A_T$ is a total area of the image and m is a mass flow of the textile fiber structure (9) per unit time.

12. Device (100) for the optical characterization of a textile fiber structure (9), with an optical sensor system (105) for detecting a plurality of pieces of information on color at different locations of the textile fiber structure (9), and

a computer (107) connected to the optical sensor system (105), wherein
the optical sensor system (105) comprises transmitting means for transmitting the detected color information to the computer (107),
the computer (107) comprises receiving means for receiving the color information transmitted by the optical sensor system (105), and
the computer (107) is adapted

to enter the captured color information in a color space (2, 2') as a scatter plot (31),
to determine a frequency density distribution (4) of the scatter plot (31),
to specify the frequency density distribution (4) numerically and
to output a signal dependent on the numerical specification.

13. Device (100) according to claim 12, wherein the computer (107) is adapted to define a characteristic region (5, 5') in the color space (2, 2') based on the frequency density distribution (4) and to numerically specify the characteristic region (5, 5') for the numerical specification of the frequency density distribution (4).

14. Device (100) according to claim 12 or 13, wherein the computer (107) is adapted to infer a change in a material composition of the textile fiber structure (9) from a change in the numerical specification of the frequency density distribution (4), and the signal output by the computer (107) is dependent on the change in the material composition.

15. Device (100) according to claim 14, wherein the computer (107) is adapted in such a way that the signal output by it is suitable for triggering at least one of the following actions: outputting a graphic to an operator, outputting a warning to an operator, outputting a recommendation for action to an operator, switching off at least one processing machine, changing a setting on at least one processing machine.

16. Device (100) according to claim 12 or 13, wherein

the device (100) additionally comprises a separation unit (109) connected to the computer (107), and
the computer (107) is adapted to recognize a foreign material (90) in the textile fiber structure (9) on the basis of the numerical specification of the frequency density distribution (4) and, when the foreign material (90) is recognized, to control a separation of the recognized foreign material (90) by the separation unit (109) by means of the signal output by it.

17. Device (100) according to claims 13 and 16, wherein the computer (107) is adapted to recognize an element of the scatter plot (31) as belonging to a foreign material (90) if the element in question lies outside the characteristic region (5, 5').

**Revendications**

1. Procédé exécuté sur ordinateur pour la caractérisation optique d'une structure de fibres textiles (9), dans lequel

plusieurs informations de couleur sont acquises en différents endroits de la structure de fibres textiles (9) par un système de capteur optique (105) ;
les informations de couleur acquises sont envoyées par le système de capteur optique (105) à un ordinateur (107) connecté au système de capteur optique, reçues et enregistrées par celui-ci,
les informations de couleur enregistrées sont saisies par l'ordinateur (107) dans un espace colorimétrique (2, 2') sous la forme d'un nuage de points (31),
**caractérisé en ce que**
l'ordinateur (107) détermine une distribution densimétrique des fréquences (4) du nuage de points (31),
l'ordinateur (107) spécifie numériquement la distribution densimétrique des fréquences (4) et
l'ordinateur (107) émet un signal dépendant de la spécification numérique.

2. Procédé selon la revendication 1, dans lequel l'espace colorimétrique (2) est un espace colorimétrique à une ou deux dimensions dans lequel l'information sur la luminosité n'est pas prise en compte.

3. Procédé selon la revendication 1 ou 2, dans lequel l'ordinateur (107) définit, sur la base de la distribution densimétrique des fréquences (4), un domaine caractéristique (5, 5') dans l'espace colorimétrique (2, 2') et spécifie numériquement le domaine caractéristique (5, 5') pour la spécification numérique de la distribution densimétrique des fréquences (4).

**4.** Procédé selon la revendication 3, dans lequel l'ordinateur (107) utilise, pour la spécification numérique du domaine caractéristique (5, 5'), une longueur, un contenu d'aire et un volume du domaine caractéristique (5, 5'), une forme géométrique du domaine caractéristique (5, 5') ou une aire limite qui délimite le domaine caractéristique (5, 5').

**5.** Procédé selon l'une des revendications précédentes, dans lequel l'ordinateur (107) déduit d'un changement de la spécification numérique de la distribution densimétrique des fréquences (4) un changement de la composition de matériau de la structure de fibres textiles (9) et le signal émis par l'ordinateur (107) dépend du changement de la composition du matériau.

**6.** Procédé selon la revendication 5, dans lequel le signal émis par l'ordinateur (107) est utilisé pour déclencher au moins une des actions suivantes : transmission d'un graphique à un opérateur, transmission d'un avertissement à un opérateur, transmission d'une recommandation d'action à un opérateur, arrêt d'au moins une machine de traitement, changement d'un réglage sur au moins une machine de traitement.

**7.** Procédé selon l'une des revendications précédentes, dans lequel l'ordinateur (107) reconnaît un matériau étranger (90) dans la structure de fibres textiles (9) sur la base de la spécification numérique de la distribution densimétrique des fréquences (4) et le signal émis par l'ordinateur commande une extraction du matériau étranger (90) reconnu hors de la structure de fibres textiles (9).

**8.** Procédé selon les revendications 3 ou 4, d'une part, et 7 d'autre part, dans lequel l'ordinateur (107) reconnaît un élément du nuage de points (31) comme faisant partie d'un matériau étranger (90) si l'élément en question se situe en dehors du domaine caractéristique (5, 5').

**9.** Procédé selon l'une des revendications précédentes, dans lequel l'ordinateur (107) corrige les informations de couleur, une valeur de seuil et/ou une plage de tolérance avec un degré de résolution qui indique dans quelle mesure la structure de fibres textiles (9) est résolue en flocons de fibres distincts.

**10.** Procédé selon la revendication 9, dans lequel le système de capteur optique (105) acquiert au moins une image de la structure de fibres textiles (9) et l'ordinateur détermine par traitement d'image, à partir de l'au moins une image, l'aire de l'image sur laquelle du matériau fibreux est visualisé,

l'ordinateur (107) détermine un flux massique de la structure de fibres textiles (9) par unité de temps et

l'ordinateur (107) calcule le taux de résolution sous forme de grandeur scalaire en calculant un quotient à partir de l'aire de l'image sur laquelle le matériau fibreux est visualisé, d'une part, et à partir du flux massique, d'autre part.

**11.** Procédé selon la revendication 9 ou 10, dans lequel l'ordinateur (107) détermine le degré de résolution selon la formule

$$ X = A_F / (A_T \cdot m) $$

où $A_F$ est l'aire de l'image sur laquelle du matériau fibreux est visualisé, $A_T$ une aire totale de l'image et m un flux massique de la structure de fibres textiles (9) par unité de temps.

**12.** Dispositif (100) pour la caractérisation optique d'une structure de fibres textiles (9), avec un système de capteur optique (105) pour capter plusieurs informations de couleur en différents endroits de la structure de fibres textiles (9) et

avec un ordinateur (107) connecté au système de capteur optique (105), dans lequel le système de capteur optique (105) comporte des moyens d'émission pour l'envoi des informations de couleur acquises à l'ordinateur (107), l'ordinateur (107) comporte des moyens de réception pour recevoir les informations de couleur émises par le système de capteur optique (105) et

l'ordinateur (107) est configuré pour

entrer les informations de couleur acquises dans un espace colorimétrique (2, 2') sous forme de nuage de points (31), déterminer une distribution densimétrique des fréquences (4) du nuage de points (31), spécifier numériquement la distribution densimétrique des fréquences (4) et émettre un signal dépendant de la spécification numérique.

**13.** Dispositif (100) selon la revendication 12, dans lequel l'ordinateur (107) est configuré pour définir un domaine caractéristique (5, 5') dans l'espace colorimétrique (2, 2') sur la base de la distribution densimétrique des fréquences (4) et spécifier numériquement le domaine caractéristique (5, 5') pour la spécification numérique de la distribution densimétrique des fréquences (4).

**14.** Dispositif (100) selon la revendication 12 ou 13, dans lequel l'ordinateur (107) est configuré pour déduire d'un changement de la spécification numérique de la distribution densimétrique des fréquences (4) un changement d'une composition du matériau de la structure de fibres textiles (9) et le signal émis par l'ordinateur (107) dépend du changement de la composition du matériau.

15. Dispositif (100) selon la revendication 14, dans lequel l'ordinateur (107) est configuré de telle façon que le signal qu'il émet peut déclencher au moins une des actions suivantes : transmission d'un graphique à un opérateur, transmission d'un avertissement à un opérateur, transmission d'une recommandation d'action à un opérateur, arrêt d'au moins une machine de traitement, changement d'un réglage sur au moins une machine de traitement.

16. Dispositif (100) selon la revendication 12 ou 13, dans lequel

le dispositif (100) inclut en outre une unité d'extraction (109) connectée à l'ordinateur (107) et l'ordinateur (107) est configuré pour détecter un matériau étranger (90) dans la structure de fibres textiles (9), sur la base de la spécification numérique de la distribution densimétrique des fréquences (4) et, si le matériau étranger est détecté (90), pour commander au moyen du signal qu'il émet une extraction du matériau étranger (90) détecté par l'unité d'extraction (109).

17. Dispositif (100) selon les revendications 13 et 16, dans lequel l'ordinateur (107) est configuré pour reconnaître un élément du nuage de points (31) comme faisant partie d'un matériau étranger (90) si l'élément en question se situe en dehors du domaine caractéristique (5, 5').

Fig. 1

Fig. 2(b)

Fig. 2(a)

Fig. 3(a)

Fig. 3(b)

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6452157 B1
- WO 2021051210 A1
- WO 2006079426 A1
- WO 2017117688 A1

**In der Beschreibung aufgeführte Nicht-Patentliteratur**

- **VON GRAF, HENNING** ; **WILRICH**. Statistische Methoden bei textilen Untersuchungen. Springer-Verlag, 1974